(19) Europäisches Patentamt
European Patent Office
Office européen des brevets

(11) Veröffentlichungsnummer: **0 044 423**
**B1**

(12) # EUROPÄISCHE PATENTSCHRIFT

(45) Veröffentlichungstag der Patentschrift: **28.09.83**

(51) Int. Cl.³: **C 07 D 249/12,** C 08 K 5/34

(21) Anmeldenummer: **81104776.0**

(22) Anmeldetag: **22.06.81**

(54) **Triazolidin-3,5-dion-Salze und ihre Verwendung zum Modifizieren oder Flammfestausrüsten von Polymeren.**

(30) Priorität: **21.07.80 DE 3027545**

(43) Veröffentlichungstag der Anmeldung: **27.01.82 Patentblatt 82/4**

(45) Bekanntmachung des Hinweises auf die Patenterteilung: **28.09.83 Patentblatt 83/39**

(84) Benannte Vertragsstaaten: **CH DE FR GB IT LI NL SE**

(56) Entgegenhaltungen:
**CHEMICAL ABSTRACTS, Vol. 63, No. 6, 13. September 1965, Columbus, Ohio, USA, G. PALAZZO »Thermal decomposition of N,N-dibenzylcarbamoyl azide« Abstract No. 6999b**
**CHEMICAL ABSTRACTS, Vol. 66, No. 22, 29. Mai 1967, Columbus, Ohio, USA, EASTMAN KODAK CO. »Photodevelopable direct-recording silver halide emulsions« Abstract No. 100194x**
**CHEMICAL ABSTRACTS, Vol. 61, No. 3, 3. August 1964, Columbus, Ohio, USA, J. BOURDAIS »Heterocyclic series II., New systheses and physiochemical properties of the 1,2,4-triazolidine-3,5-diones« Abstract No. 3094th**

(73) Patentinhaber: **BAYER AG, Zentralbereich Patente, Marken und Lizenzen, D-5090 Leverkusen 1, Bayerwerk (DE)**

(72) Erfinder: **Rottmaier, Ludwig, Bergstrasse 85, D-5068 Odenthal (DE)**
Erfinder: **Merten, Rudolf, Dr., Berta-von-Suttner-Strasse 55, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Triazolidin-3,5-dion-Salze und ihre Verwendung zum Modifizieren oder Flammfestausrüsten von Polymeren

Die vorliegende Erfindung betrifft neue, aus organischen Aminen hergestellte Triazolidin-3,5-dion-Salze, welche mindestens einmal die Gruppierung der allgemeinen Formel I

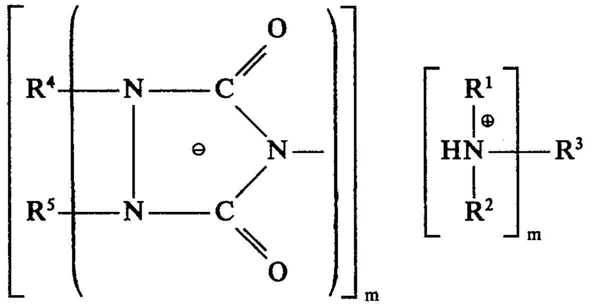

I

enthalten, worin

$R^1$ und $R^2$ gleich oder verschieden, für Wasserstoff, einen gegebenenfalls mit Halogen, Hydroxy, $C_1-C_{10}$ Alkoxy oder $C_2-C_{10}$ Alkoxycarbonyl substituierten aliphatischen $C_1-C_{10}$, cycloaliphatischen $C_4-C_{10}$, araliphatischen $C_7-C_{17}$ oder aromatischen $C_6-C_{10}$ Rest stehen.

$R^3$ für einen gegebenenfalls mit Halogen, Cyano, Nitro, Hydroxy, Amino, $C_1-C_{10}$ Alkylamino, $C_1-C_{10}$ Dialkylamino, $C_1-C_{10}$ Alkoxy, $C_2-C_{10}$ Alkoxycarbonyl substituierten m-wertigen aliphatischen $C_1-C_{20}$, vorzugsweise $C_1-C_{10}$; cycloaliphatischen $C_4-C_{15}$, vorzugsweise $C_5-C_{10}$ araliphatischen $C_7-C_{17}$, vorzugsweise $C_7-C_{10}$, aromatischen $C_6-C_{15}$, vorzugsweise $C_6-C_{10}$ und einen N, O oder S im Ring enthaltenden Aryl- oder $C_2-C_{12}$ Cycloalkylrest,

$R^4$ und $R^5$, gleich oder verschieden, für Wasserstoff, einen gegebenenfalls mit Halogen, Cyano, Hydroxy, $C_1-C_{10}$ Alkoxy und $C_2-C_{10}$ Alkoxycarbonyl substituierten aliphatischen $C_1-C_{20}$, vorzugsweise $C_1-C_{10}$ araliphatischen $C_7-C_{17}$, vorzugsweise $C_7-C_{10}$, aromatischen $C_6-C_{15}$, vorzugsweise $C_6-C_{10}$ oder einen gegebenenfalls mit Halogen substituierten $C_2-C_{20}$ vorzugsweise $C_2-C_{10}$ Alkylcarbonylrest und

m für eine ganze Zahl von 1 bis 5, vorzugsweise 1 bis 3, stehen.

Die Reste $R^1$ und/oder $R^2$ und/oder $R^3$ können auch als Glieder eines cyclischen, gegebenenfalls noch N, O oder S im Ring enthaltenden $C_2-C_{10}$-Alkylrestes miteinander verknüpft sein.

Eine besondere Variante der erfindungsgemäßen Triazolidin-3,5-dion-Salze sind solche der allgemeinen Formel Ia

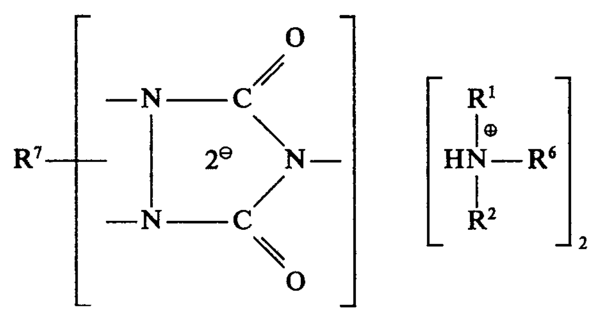

Ia

worin

$R^1$ und $R^2$ die oben angegebene Bedeutung haben,

$R^6$ für einen gegebenenfalls mit Halogen, Cyano, Nitro, Hydroxy, Amino, $C_1-C_{10}$ Alkyl- oder Dialkylamino, $C_1-C_{10}$ Alkoxy, $C_2-C_{10}$ Alkoxycarbonyl substituierten aliphatischen $C_1-C_{20}$, vorzugsweise $C_1-C_{10}$ cycloaliphatischen $C_4-C_{15}$, vorzugsweise $C_5-C_{10}$ araliphatischen $C_7-C_{17}$, vorzugsweise $C_7-C_{10}$ aromatischen $C_6-C_{15}$, vorzugsweise $C_6-C_{10}$ und einen N, O oder S im Ring enthaltenden Aryl- oder $C_2-C_{12}$ Cycloalkylrest und

$R^7$ die Bedeutung von $R^4$ oder $R^5$ hat.

Weitere erfindungsgemäße Triazolidin-3,5-dion-Salze sind solche der allgemeinen Formel II

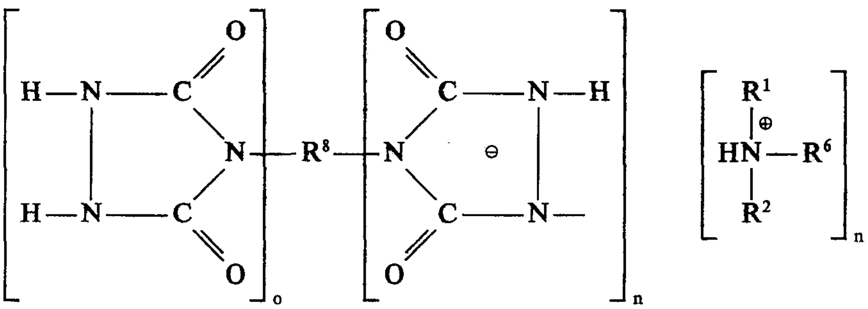

II

worin

$R^1$, $R^2$ und $R^6$ die in der allgemeinen Formel Ia beschriebene Bedeutung haben,

$R^8$ für einen gegebenenfalls mit Halogen, Hydroxy, $C_1-C_{10}$ Alkoxy, $C_1-C_{10}$ Alkyl- bzw. Dialkylamino oder $C_2-C_{10}$ Alkoxycarbonyl substituierten (n+o)-wertigen aliphatischen $C_1-C_{20}$, vorzugsweise $C_1-C_{10}$ cycloaliphatischen $C_4-C_{15}$, vorzugsweise $C_5-C_{10}$ araliphatischen $C_7-C_{20}$, vorzugsweise $C_7-C_{10}$ oder aromatischen $C_6-C_{15}$, vorzugsweise $C_6-C_{10}$ Rest und n für eine ganze Zahl von 1 bis 5, vorzugsweise 1, bis 3, o für 0 oder eine ganze Zahl von 1 bis 4 stehen, wobei n und o nicht größer als 5, vorzugsweise nicht größer als 3 sein darf.

Die erfindungsgemäßen, aus organischen Verbindungen hergestellten Triazolidin-3,5-dion-Salze, welche eine Gruppierung der allgemeinen Formel I enthalten, sind neu und werden erhalten, indem man Triazolidin-3,5-dione, welche die Struktur der allgemeinen Formel III

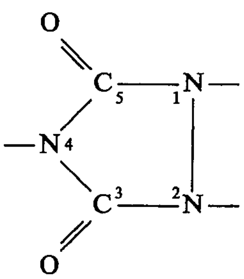

III

mindestens einmal enthalten und welche mindestens ein gebundenes Wasserstoffatom in 1- oder 2- oder 4-Stellung enthalten müssen, mit Aminen der allgemeinen Formel IV

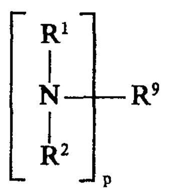

IV

worin

$R^1$ und $R^2$ die oben beschriebene Bedeutung haben,

$R^9$ für einen gegebenenfalls mit Halogen, Cyano, Nitro, Hydroxy, Amino, $C_1-C_{10}$ Alkylamino, $C_1-C_{10}$ Dialkylamino, $C_1-C_{10}$ Alkoxy, $C_2-C_{10}$ Alkoxycarbonyl substituierten aliphatischen $C_1-C_{20}$, cycloaliphatischen $C_4-C_{15}$, araliphatischen $C_7-C_{17}$, aromatischen $C_6-C_{15}$ oder einen Heteroatome wie N, O oder S im Ring enthaltenden Aryl- oder $C_2-C_{12}$-Cycloalkylrest und

p für eine ganze Zahl von 1 bis 5, vorzugsweise 1 bis 3, stehen, in Wasser oder in organischen Lösungsmitteln und/oder in Mischungen aus Wasser und mit Wasser mischbare organische Lösungsmittel bei Temperaturen von 0 bis 150° C umsetzt.

Die zur Herstellung der neuen Triazolidin-3,5-dion-Salze verwendeten Triazolidin-3,5-dione sind teilweise aus der Literatur bekannt und können durch Reaktion von Hydrazincarbonsäureester mit Monoisocyanaten und nachfolgender Cyclisierung oder aus Hydrazin-N,N'-dicarbonsäurediamid durch Erhitzen auf 200° C, z. B. gemäß deutscher Patentanmeldung Nr. 2 947 619.5 erhalten werden. Entsprechende Oligo-triazolidin-3,5-dione können durch Reaktion von primären Polyaminen mit Hydrazin-N,N'-dicarbonsäurediamid bzw. mit Triazolidin-3,5-dion oder durch Reaktion von Polyisocyanaten mit Semicarbazid bzw. Hydrazincarbonsäureester, wie in den deutschen Patentanmeldungen P 3 027 611.0 und P 3 027 612.1 beschrieben, hergestellt werden.

Als Beispiele seien genannt: Triazolidin-3,5-dion, 1-, 2-, 4-Methyl-triazolidin-3,5-dion, 1-, 2-,

4-Ethyl-triazolidin-3,5-dion, 4-Cyclohexyl-triazolidin-3,5-dion, 4-(2-Hydroxyethyl)-triazolidin-3,5-dion, 4-(2-Chlorethyl)-triazolidin-3,5-dion, 1-, 2-, 4-Phenyl-triazolidin-3,5-dion, 1,2-Dimethyl-, 1,2-Diethyl-, 1,2-Diphenyl-triazolidin-3,5-dion, 4-(p-Hydroxyphenyl)-triazolidin-3,5-dion, 1,6-Bis(1,2,4-triazolidin-3,5-dion-4-yl)-hexan und 1,4-Bis-(triazolidin-3,5-dion-4-yl)-butan. Besonders bevorzugt wird 1,2,4-Triazolidin-3,5-dion verwendet.

Zur Herstellung der Triazolidin-3,5-dion-Salze mit der Gruppierung der allgemeinen Formel I werden vorzugsweise aliphatische, cycloaliphatische, araliphatische aromatische und Heteroatome enthaltende cyclische Amine der oben angegebenen Formel V verwendet. Gemäß dieser Formel sind die Reste $R^1$ und $R^2$ bevorzugt aliphatische $C_1-C_{10}$, cycloaliphatische $C_4-C_{10}$, araliphatische $C_7-C_{17}$ oder aromatische $C_6-C_{10}$ Reste, welche gegebenenfalls mit Halogen, Hydroxy oder $C_1-C_{10}$ Alkoxy, substituiert sein können.

Der Rest $R^9$ ist bevorzugt ein p-wertiger aliphatischer $C_1-C_{20}$, cycloaliphatischer $C_4-C_{15}$, araliphatischer $C_7-C_{17}$, aromatischer $C_6-C_{15}$ und Heteroatome wie N, O oder S im Ring enthaltender Aryl- oder $C_2-C_{12}$-Cycloalkylrest. Diese Reste können mit Hydroxy, Amino, $C_1-C_{10}$ Alkoxy, $C_1-C_{10}$ Alkyl- bzw. Dialkylamino, und $C_2-C_{20}$-Alkoxycarbonyl substituiert sein.

Die Reste $R^1$ und/oder $R^2$ und/oder $R^9$ können auch als Glieder eines cyclischen, gegebenenfalls noch N, O oder S im Ring enthaltenden $C_2-C_{10}$-Alkylenrestes miteinander verknüpft sein.

Insbesondere bedeutet der Rest $R^9$ 1,3,5-Triazin und daraus abgeleitete Derivate.

Als Beispiele seien genannt: Aliphatische Amine wie z. B. Methylamin, Ethylamin, Propylamin, Butylamin, Hexylamin, Dodecylamin, Hexadecylamin, Stearylamin, Allylamin, Dimethylamin, Diethylamin, Dibutylamin, Bis-(2-ethylhexyl)-amin, 1,2-Diaminoethan, N,N'-Dimethyl-ethylen-diamin, 1,2-Diaminopropan, Dipropylentetramin, 1,4-Diaminobutan, 1,6-Diaminohexan, 2,5-Diamino-2,5-dimethylhexan, 2,2,4-, 2,4,4-Trimethyl-hexamethylendiamin, Trimethylamin, Triethylamin und Tributylamin, aliphatische Aminoalkohole wie z. B. Ethanolamin, 2-Methoxy-ethylamin, Isopropanolamin, Triethanolamin, Triisopropanolamin, 3-Hydroxy-, 3-Methoxypropylamin und 2-Amino-2-methyl-propandiol, aliphatische Aminocarbonsäureester wie z. B. Aminoessigsäuremethylester, 3-Aminopropionsäureisobutylester, ε-Aminocapronsäuremethylester und N-Methyl-asparaginsäuredimethylester, cycloaliphatische Amine und Aminoalkohole wie z. B. Cyclohexylamin, N-Methyl-cyclohexylamin, Isophorondiamin, 1,4-Diaminocyclohexan, N-(2-Hydroxyethyl)- und N-(2-Hydroxypropyl)-cyclohexylamin, aromatische Amine wie z. B. Anilin, Methylanilin, Diphenylamin, (2-Hydroxyethyl)-anilin, Anilinoessigsäuremethylester, Chloranilin, 4-Nitranilin, p-Aminophenol, 2-Aminotoluol, 1-Aminonaphthalin, Benzylamin, 4-Amino-benzoesäure-ethylester, 1,2-, 1,3-, 1,4-Diamino-benzol, 4,4'-Diamino-diphenylmethan, -propan-2, -ether und 1,5-Diaminonaphthalin, heterocyclische Amine wie z. B. Pyrrolidin, Piperidin, Pyridin, Carbazol, Piperazin, Pyrazol, Imidazol, Morpholin, Melamin, 1,2-Bis(4,6-diamino-1,3,5-triazin-2-yl)ethan, 1,4-Bis-(4,6-diamino-1,3,5-triazin-2-yl)-butan, 1,6-Bis- und 1,3,6-Tris(4,6-diamino-1,3,5-triazin-2-yl)hexan.

Die Reaktion der Triazolidin-3,5-dione mit den Aminen wird normalerweise mit äquivalenten Mengen an Aminen durchgeführt, d. h. ein Mol Triazolidin-3,5-dion wird mit einer Aminogruppe zur Reaktion gebracht. Werden die Triazolidin-3,5-dione jedoch mit Polyaminen umgesetzt, so können hierbei auch Salze mit freien Aminogruppen erhalten werden, wenn z. B. ein Mol 1,2,4-Triazolidin-3,5-dion mit 1 Mol Polyamin umgesetzt wird. Es können somit für jeden Einsatzzweck fast beliebig modifizierbare Triazolidin-3,5-dion-Salze erhalten werden.

Die Reaktion zwischen den Triazolidin-3,5-dionen und den Aminen der allgemeinen Formel IV kann in der Schmelze oder in Lösungsmitteln durchgeführt werden. Vorzugsweise wird die Reaktion jedoch in Lösungsmitteln durchgeführt. Dabei kann auch eine der Reaktionskomponente, normalerweise das Amin, im Überschuß eingesetzt, als Lösungsmittel verwendet werden.

Ferner können mit den Reaktionskomponenten nicht reagierende, insbesondere polare, Lösungsmittel verwendet werden, sofern die Reaktionsprodukte in gelöster oder kristalliner Form vorliegen.

Vorteilhafterweise wird die Reaktion zwischen den Triazolidin-3,5-dionen und den Aminen jedoch in wäßrigem Medium und/oder in mit Wasser mischbaren Lösungsmitteln durchgeführt. Als Beispiele für diese mit Wasser mischbaren Lösungsmittel seien genannt: Alkohole wie Methanol, Ethanol, Isopropanol, Glykolmonomethylether und Glykol, cyclische Ether wie Tetrahydrofuran und Dioxan, Alkyl- und Dialkylamide wie Dimethylformamid, Dimethylacetamid und N-Methylpyrrolidon. Selbstverständlich können auch Mischungen dieser Lösungsmittel bzw. Mischungen mit Wasser verwendet werden. Auch können nach beendeter Reaktion, evtl. zur besseren Aufarbeitung der erhaltenen Triazolidin-3,5-dion-Salze, weitere Lösungsmittel wie z. B. aliphatische und aromatische Kohlenwasserstoffe (Ligroin, Cyclohexan, Toluol, Xylol), chlorierte Kohlenwasserstoffe (Methylenchlorid, Chloroform, Chlorbenzol) und Ester (Essigsäureethyl-, -butylester) zugesetzt werden. Die Salze werden nach üblichen bekannten Methoden isoliert.

Insbesondere findet bei der Reaktion zwischen den Triazolidin-3,5-dionen und den Aminen Wasser als Lösungsmittel Verwendung, da häufig die gewünschte Verbindung in praktisch reiner Form beim Abkühlen aus der wäßrigen Lösung auskristallisiert.

Die Reaktion zwischen den Triazolidin-3,5-dionen und den Aminen wird bei 0 bis 150°C, vorzugsweise bei 30 bis 130°C, gegebenenfalls unter erhöhtem Druck, durchgeführt.

Die Reaktionszeiten liegen im allgemeinen zwischen einigen Minuten und mehreren Stunden, können in besonderen Fällen jedoch auch darüber liegen. Durch entsprechende Wahl der Reaktionsbedingungen, z. B. Druck, werden kürzere Reaktionszeiten erreicht.

Die Triazolidin-3,5-dion-Salze mit der Gruppierung der allgemeinen Formel I sind wertvolle Hilfsstoffe zur Modifizierung von polymeren Substanzen. Sie können zur Herstellung und Modifizierung von Polyurethanen oder zur Flammschutzausrüstung von Polymeren, z. B. Polyamiden, eingesetzt werden.

In den nachfolgenden Beispielen bedeuten die Prozente Gewichtsprozente und Teile Gewichtsteile.

## Beispiel 1

Zu einer Lösung aus 101 g Triazolidin-3,5-dion in 300 g Wasser werden bei 95° C 99 g Cyclohexylamin in einer Stunde zugetropft. Die klare Lösung wird 3 Stunden bei 95° C gerührt und auf Raumtemperatur abgekühlt. Der entstandene Niederschlag wird abgesaugt, mit Wasser gewaschen und getrocknet (Ausbeute: 66 g). Aus der Mutterlauge kristallisierte nach Zugabe von Aceton weiteres Salz aus, welches laut IR-Spektrum mit dem 1. Niederschlag identisch ist (Ausbeute: 95 g). IR-Spektrum und Elementaranalyse bestätigen das Cyclohexylamin-triazolidin-3,5-dion-Salz.

| | | | |
|---|---|---|---|
| berechnet für $C_8H_{16}N_4O_2$: | C = 48%; | H = 8%; | N = 28% |
| gefunden: | C = 47,5%; | H = 8,1%; | N = 27,7% |

## Beispiel 2

Zu einer Lösung von 101 g Triazolidin-3,5-dion in 200 g Wasser werden bei 95° C 107 g Benzylamin getropft. Die erhaltene Lösung wird 1 Stunde bei 95° C gerührt, mit Ethanol versetzt und auf Raumtemperatur abgekühlt. Nach dem Absaugen und waschen mit Ethanol werden 164 g Salz erhalten, dessen Formel durch das IR-Spektrum und der Elementaranalyse bestätigt wird.

| | | | |
|---|---|---|---|
| berechnet für $C_9H_{12}N_4O_2$: | C = 51,9%; | H = 5,77%; | N = 26,9% |
| gefunden: | C = 51,6%; | H = 5,9%; | N = 26,9% |

## Beispiel 3

Eine Lösung von 116 g Hexamethylendiamin in Methanol wird bei 90° C zu 202 g Triazolidin-3,5-dion in 300 g Wasser so zugetropft, daß das Methanol abdestilliert. Nach zweistündiger Reaktion bei 90° C wird abgekühlt, der erhaltene Niederschlag abgesaugt und bei 50° C im Vakuum getrocknet (Ausbeute: 229 g). Die Bestimmung des Wassergehaltes nach Fischer ergab 10,5% Wasser, d. h. ein Mol Diamin-Salz kristallisiert mit 2 Mol Kristallwasser. IR-Spektrum und Elementaranalyse einer bei 120° C im Vakuum getrockneten Probe bestätigen die angenommene Struktur.

| | | | |
|---|---|---|---|
| berechnet für $C_{14}H_{28}N_8O_4$: | C = 45,2%; | H = 7,53%; | N = 30,1% |
| gefunden: | C = 44,8%; | H = 7,7%; | N = 29,7% |

## Beispiel 4

Zu einer Lösung von 202 g Triazolidin-3,5-dion in 600 ml N-Methylpyrrolidon werden bei 35° C in einer Stunde 170 g Isophorondiamin zugetropft. Die erhaltene Suspension wird mit 1 l Ethanol verdünnt und abgesaugt. Nach dem Nachwaschen mit Ethanol und Chloroform wird das stark hydroskopische Produkt sofort im Vakuum getrocknet (Ausbeute: 315 g). IR-Spektrum und Elementaranalyse bestätigen die angenommene Struktur.

| | | | |
|---|---|---|---|
| berechnet für $C_{14}H_{28}N_8O_4$: | C = 45,2%; | H = 7,53%; | N = 30,1% |
| gefunden: | C = 44,8%; | H = 7,7%; | N = 29,7% |

## Beispiel 5

202 g Triazolidin-3,5-dion und 300 g Wasser werden unter Rühren auf 95° C erwärmt und langsam mit 60 g 1,2-Diaminoethan versetzt. Die entstandene Lösung wird abgekühlt, mit 800 ml Isopropanol versetzt, der erhaltene Niederschlag abgesaugt und mit Isopropanol gewaschen. Nach dem Trocknen

bei 50°C im Vakuum werden 191 g Salz erhalten, dessen Struktur mittels IR-Spektrum und Elementaranalyse bestätigt wurde.

| | | | |
|---|---|---|---|
| berechnet für $C_6H_{14}N_8O_4$: | C = 27,5%; | H = 5,35%; | N = 42,7% |
| gefunden: | C = 27,6%; | H = 5,5%; | N = 42,3% |

Beispiel 6

a) 4,41 kg Melamin werden in 105 l Wasser durch Erwärmen auf 95°C gelöst und mit 3,73 kg eines technischen, 94,7%igen Triazolidin-3,5-dions (bestimmt mittels Titration mit n/10 Natronlauge gegen Phenolphthalein) versetzt. Die erhaltene Suspension wird zur Vervollständigung der Reaktion noch 3 Stunden bei 95°C nachgerührt, auf Raumtemperatur abgekühlt, abgesaugt, mit Wasser gewaschen und bei 85°C im Vakuum bei 30 mbar getrocknet (Ausbeute 6,9 kg = 90,4% der Theorie. IR-Spektrum und Elementaranalyse bestätigen die angenommene Salzstruktur.

| | | | |
|---|---|---|---|
| berechnet für $C_5H_9N_9O_2$: | C = 26,4%; | H = 3,96%; | N = 55,5% |
| gefunden: | C = 26,3%; | H = 3,9%; | N = 55,1% |

b) 95 Gew.-% Polyamid-6 mit einer relativen Viskosität (gemessen an einer Lösung aus 1 g Polyamid in 100 ml m-Kresol bei 25°C) von 3,01 werden in einem Doppelwellenextruder bei den für Polyamid-6 üblichen Bedingungen mit 5 Gew.-% des nach a) hergestellten Salzes in der Schmelze vermischt. Der abgezogene Strang wird gekühlt, granuliert und getrocknet. Anschließend wird das Granulat auf einer Spritzgießmaschine A 270 der Firma Arburg zu Probekörpern der Abmessung 127 × 12,7 × 1,6 mm gespritzt.

Diese Probekörper werden 24 Stunden bei 23°C und 50% rel. Luftfeuchte gelagert und ergaben bei der Brandprüfung gemäß Underwriter's Laboratories (UL) Subject 94 »Vertical Burning Test for Classifying Materials« eine Brandfestigkeit von VO.

Beispiel 7

Zu einer Mischung von 101 g Triazolidin-3,5-dion und 100 g Wasser werden bei 80°C 31 g Methylamin als 38%ige wäßrige Lösung zugetropft. Die entstandene klare Lösung wird 2 Stunden bei 80°C gerührt und im Vakuum teilweise eingeengt. Der verbliebene Rückstand (177 g) wird mit 150 ml Methanol versetzt und abgekühlt. Der auskristallisierte Niederschlag wird abgesaugt, mit Methanol gewaschen und getrocknet (Ausbeute: 81 g). IR-Spektrum und Elementaranalyse bestätigen die angenommene Struktur.

| | | | |
|---|---|---|---|
| berechnet für $C_3H_8N_4O_3$: | C = 27,3%; | H = 6,06%; | N = 40,4% |
| gefunden: | C = 27,1%; | H = 6,2%; | N = 40,1% |

Beispiel 8

Zu einer Mischung von 1010 g Triazolidin-3,5-dion in 1 kg Wasser werden bei 95°C 1330 g aufgeschmolzenes Diisopropanolamin in 30 Minuten zugetropft. Die entstandene Lösung wird 1 Stunde bei 95°C gerührt und im Vakuum eingeengt. Der Rückstand von 2,7 kg wird in 3,5 l Ethanol heiß gelöst und durch Abkühlen zum Kristallisieren gebracht. Der Niederschlag wird abgesaugt, mit Ethanol gewaschen und getrocknet (Ausbeute: 1480 g). IR-Spektrum und Elementaranalyse bestätigen die angenommene Struktur.

| | | | |
|---|---|---|---|
| berechnet für $C_8H_{18}H_4O_4$: | C = 41,1%; | H = 7,69%; | N = 23,9% |
| gefunden: | C = 40,9%; | H = 7,8%; | N = 23,7% |

Beispiel 9

Zu 50,5 g Triazolidin-3,5-dion in 50 g Wasser werden bei 85°C 50,5 g Triethylamin getropft. Die entstandene Lösung wird 30 Minuten bei 80°C gerührt, auf Raumtemperatur abgekühlt und der auskristallisierte Niederschlag durch Absaugen isoliert. Nach dem Trocknen bei 50°C im Vakuum werden 57 g Salz vom Fp. = 69–72°C erhalten, welches 2 Mol Kristallwasser enthält.

| | | | | |
|---|---|---|---|---|
| berechnet für $C_8H_{18}N_4O_2 \cdot 2\ H_2O$: | C = 40,3%; | H = 9,25%; | N = 23,5%; | $H_2O$ = 15,1% |
| gefunden: | C = 40,0%; | H = 9,1%; | N = 23,4%; | $H_2O$ = 15,2% |

### Beispiel 10

50,5 g Triazolidin-3,5-dion und 50 g Wasser werden bei 95°C mit einer wäßrigen Lösung von 34 g Imidazol versetzt. Die entstandene, klare Lösung wird 2 Stunden bei 95°C gerührt und am Rotationsverdampfer bei 60°C eingeengt. Der Rückstand wird mit 150 ml Ethanol versetzt und die erhaltenen Kristalle durch Absaugen isoliert. Nach dem Trocknen bei 50°C im Vakuum werden 66,5 g Salz erhalten, dessen Struktur durch IR-Spektrum und Elementaranalyse bestätigt wird.

| | | | |
|---|---|---|---|
| berechnet für $C_5H_7N_5O_2$: | C = 35,4%; | H = 4,13%; | N = 41,3% |
| gefunden: | C = 35,6%; | H = 3,9%; | N = 40,9% |

### Beispiel 11

Zu einer Mischung von 35,4 g 4-Phenyl-triazolidin-3,5-dion in 50 g $H_2O$ wird bei 75°C eine Lösung von 21,8 g p-Aminophenol in 150 ml Methanol in 30 Minuten zugetropft. Die Temperatur der entstandenen Lösung wird in 2 Stunden auf 85°C gesteigert, wobei ein Teil des Methanols abdestilliert. Nach dem Abkühlen werden die erhaltenen Kristalle abgesaugt und getrocknet (Ausbeute 45 g). Die Struktur des erhaltenen Salzes, welches aus Wasser umkristallisiert werden kann, wird durch das IE-Spektrum und der Elementaranalyse bestätigt.

| | | | |
|---|---|---|---|
| berechnet für $C_{14}H_{14}N_4O_3$: | C = 58,7%; | H = 4,89%; | N = 19,6% |
| gefunden: | C = 59,0%; | H = 4,8%; | N = 19,7% |

### Beispiel 12

Zu einer Mischung von 27,6 g 1,4-Bis(4,6-diamino-1,3,5-triazin-2-yl)-butan in 1 kg Wasser werden bei 95°C 10,1 g Triazolidin-3,5-dion zugesetzt. Zur Vervollständigung der Reaktion wird noch 3 Stunden bei Rückfluß gehalten, sodann bei 80°C abgesaugt und mit Wasser nachgewaschen. Es werden nach dem Trocknen bei 80°C und 30 mbar 31 g Salz erhalten, dessen Struktur durch das IR-Spektrum und die Elementaranalyse bestätigt wird.

| | | | |
|---|---|---|---|
| berechnet für $C_{12}H_{19}N_{13}O_2$: | C = 38,2%; | H = 5,03%; | N = 48,2% |
| gefunden: | C = 38,0%; | H = 5,1%; | N = 47,9% |

### Beispiel 13

Beispiel 12 wurde mit der doppelten Menge, also mit 20,2 g Triazolidin-3,5-dion wiederholt. Es entstanden 37 g Salz, dessen Struktur durch das IR-Spektrum und der Elementaranalyse bestätigt wird.

| | | | |
|---|---|---|---|
| berechnet für $C_{14}H_{22}N_{16}O_4$: | C = 35,2%; | H = 4,6%; | N = 46,8% |
| gefunden: | C = 35,3%; | H = 4,8%; | N = 46,5% |

**Patentansprüche**

1. Triazolidin-3,5-dion-Salze nach der allgemeinen Formel I

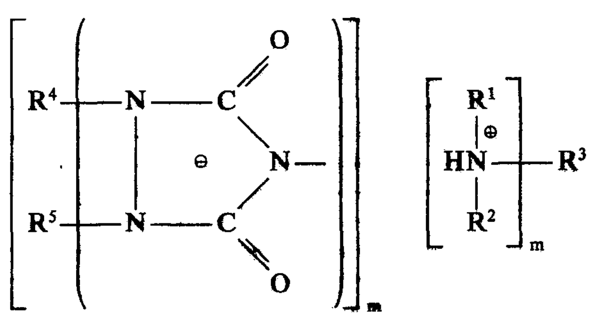

I

worin

$R^1$ und $R^2$, gleich oder verschieden, für Wasserstoff, einen gegebenenfalls mit Halogen, Hydroxy, $C_1-C_{10}$ Alkoxy oder $C_2-C_{10}$ Alkoxycarbonyl substituierten aliphatischen $C_1-C_{10}$, cycloaliphatischen $C_4-C_{10}$, araliphatischen $C_7-C_{17}$ oder aromatischen $C_6-C_{10}$ Rest stehen;

$R^3$ für einen m-wertigen, gegebenenfalls mit Halogen, Cyano, Nitro, Hydroxy, Amino, $C_1-C_{10}$ Alkyl- bzw. Dialkylamino, $C_1-C_{10}$ Alkoxy, $C_2-C_{10}$ Alkoxycarbonyl substituierten aliphatischen $C_1-C_{20}$, cycloaliphatischen $C_4-C_{15}$, araliphatischen $C_7-C_{17}$, aromatischen $C_6-C_{15}$ oder N, O und S enthaltenden $C_2-C_{12}$ Cycloalkyl- oder Arylrest, wobei die Reste $R^1$ und/oder $R^3$ auch als Glieder eines cyclischen, gegebenenfalls noch mit N, O oder S im Ring enthaltenden $C_2-C_{10}$ Alkylenrestes miteinander verknüpft sein können,

$R^4$ und $R^5$ gleich oder verschieden, für Wasserstoff, einen gegebenenfalls mit Halogen, Cyano, Hydroxy, $C_1-C_{10}$ Alkoxy und $C_2-C_{10}$ Alkoxycarbonyl substituierten aliphatischen $C_1-C_{20}$, araliphatischen $C_7-C_{17}$, aromatischen $C_6-C_{15}$ oder einen gegebenenfalls mit Halogen substituierten $C_2-C_{20}$ Alkylcarbonylrest und

m für eine ganze Zahl von 1 bis 5

stehen.

2. Triazolidin-3,5-dion-Salze gemäß Anspruch 1, dadurch gekennzeichnet, daß $R^4$ und $R^5$ Wasserstoff bedeuten.

3. Triazolidin-3,5-dion-Salze nach Anspruch 1 der allgemeinen Formel Ia

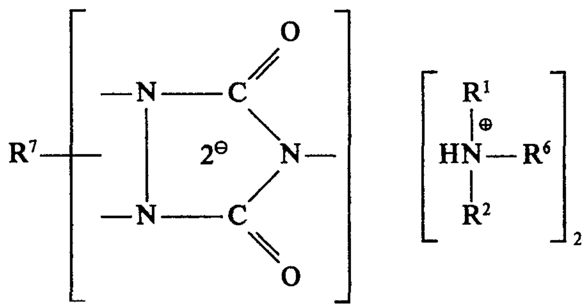

Ia

worin

$R^1$ und $R^2$ die im Anspruch 1 beschriebene Bedeutung haben,

$R^6$ für einen gegebenenfalls mit Halogen, Cyano, Nitro, Hydroxy, Amino, $C_1-C_{20}$ Alkyl- oder Dialkylamino, $C_1-C_{10}$ Alkoxy, $C_2-C_{10}$ Alkoxycarbonyl substituierten aliphatischen $C_1-C_{20}$, cycloaliphatischen $C_4-C_{15}$, araliphatischen $C_7-C_{17}$, aromatischen $C_6-C_{15}$ und Heteroatome wie N, O oder S im Ring enthaltenden Aryl- oder $C_2-C_{12}$ Cycloalkylrest stehen und

$R^7$ die Bedeutung von $R^4$ oder $R^5$ hat.

4. Triazolidin-3,5-dion-Salze nach Anspruch 3, dadurch gekennzeichnet, daß $R^7$ Wasserstoff bedeutet.

5. Triazolidin-3,5-dion-Salze nach Anspruch 1 der allgemeinen Formel II

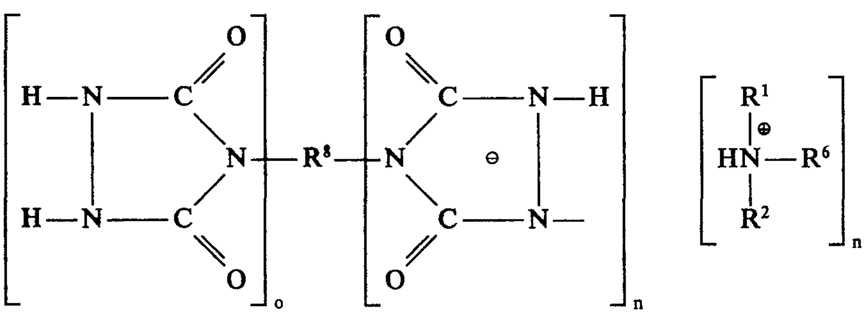

II

worin

$R^1$, $R^2$ und $R^6$ die im Anspruch 4 angegebene Bedeutung haben,

$R^8$ für einen gegebenenfalls mit Halogen, Hydroxy, $C_1-C_{10}$ Alkoxy, $C_1-C_{10}$ Alkyl bzw. Dialkylamino oder $C_2-C_{10}$ Alkoxycarbonyl substituierten (n+o)-wertigen aliphatischen $C_1-C_{20}$ cycloaliphatischen $C_4-C_{15}$, araliphatischen $C_7-C_{20}$ oder aromatischen $C_6-C_{15}$ Rest,

n für eine ganze Zahl von 1 bis 5,

o für 0 oder eine ganze Zahl von 1 bis 4 stehen, wobei n + o nicht größer als 5 sein darf.

6. Verfahren zur Herstellung von Triazolidin-3,5-dion-Salzen nach Ansprüchen 1 bis 5, dadurch gekennzeichnet, daß Triazolidin-3,5-dione, welche die Struktur der allgemeinen Formel III

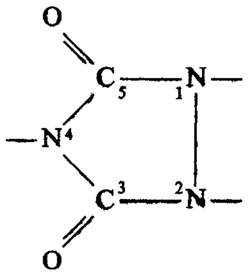

III

mindestens einmal enthalten und welche mindestens ein in 1-, 2-, oder 4-Stellung gebundenes Wasserstoffatom enthalten müssen, mit Aminen der allgemeinen Formel IV

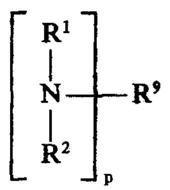

IV

worin

$R^1$ und $R^2$ die im Anspruch 1 beschriebene Bedeutung haben,

$R^9$ für einen p-wertigen, gegebenenfalls mit Halogen, Cyano, Nitro, Hydroxy, Amino, $C_1-C_{10}$ Alkylamino, Dialkylamino, $C_1-C_{10}$ Alkoxy, $C_2-C_{10}$ Alkoxycarbonyl substituierten aliphatischen $C_1-C_{20}$, cycloaliphatischen $C_4-C_{15}$, araliphatischen $C_7-C_{17}$, aromatischen $C_6-C_{15}$ oder einen Heteroatome wie N, O oder S im Ring enthaltenden Aryl- oder $C_2-C_{12}$ Cycloalkylrest,

p für eine ganze Zahl von 1 bis 5 stehen gegebenenfalls in Wasser und/oder in organischen Lösungsmitteln bei Temperaturen von 0 bis 150° C umsetzt.

7. Verwendung der Triazolidin-3,5-dion-Salze nach Ansprüchen 1 bis 6 zum Modifizieren von Polymeren.

8. Verwendung der Triazolidin-3,5-dion-Salze nach Ansprüchen 1 bis 6, zur Flammfestausrüstung von Polymeren.

**Claims**

1. Triazolidine-3,5-dione salts according to the general formula I

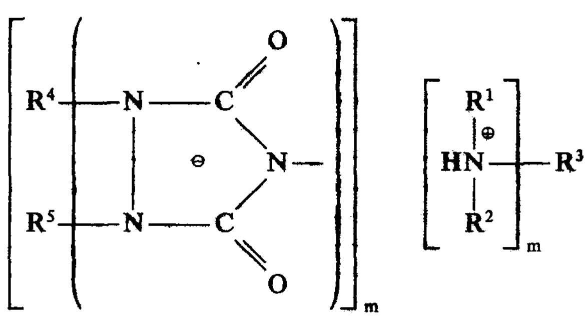

I

wherein

$R^1$ and $R^2$ are identical or different and represent hydrogen, an aliphatic $C_1-C_{10}$, cycloaliphatic $C_4-C_{10}$, araliphatic $C_7-C_{17}$ or aromatic $C_6-C_{10}$ radical optionally substituted with halogen, hydroxyl, $C_1-C_{10}$ alkoxy or $C_2-C_{10}$ alkoxycarbonyl;

$R^3$ represents an m-valent aliphatic $C_1-C_{20}$, cycloaliphatic $C_4-C_{15}$, araliphatic $C_7-C_{17}$, or aromatic $C_6-C_{15}$ radical, or a $C_2-C_{12}$ cycloalkyl or aryl radical containing N, O and S, which radicals are optionally substituted with halogen, cyano, nitro, hydroxyl, amino, $C_1-C_{10}$ alkyl- or dialkylamino, $C_1-C_{10}$ alkoxy or $C_2-C_{10}$ alkoxycarbonyl, it being possible for the radicals $R^1$ and/or $R^3$ also to be linked to one another as members of a cyclic $C_2-C_{10}$ alkylene radical optionally also containing N, O or S in the ring,

$R^4$ and $R^5$ are identical or different and represent hydrogen, an aliphatic $C_1-C_{20}$, araliphatic $C_7-C_{17}$ or aromatic $C_6-C_{15}$ radical optionally substituted with halogen, cyano, hydroxyl, $C_1-C_{10}$ alkoxy and $C_2-C_{10}$ alkoxycarbonyl or a $C_2-C_{20}$ alkylcarbonyl radical optionally substituted with halogen and

m represents an integer from 1 to 5.

2. Triazolidine-3,5-dione salts according to Claim 1, characterised in that $R^4$ and $R^5$ denote hydrogen.

3. Triazolidine-3,5-dione salts according to Claim 1 of the general formula Ia

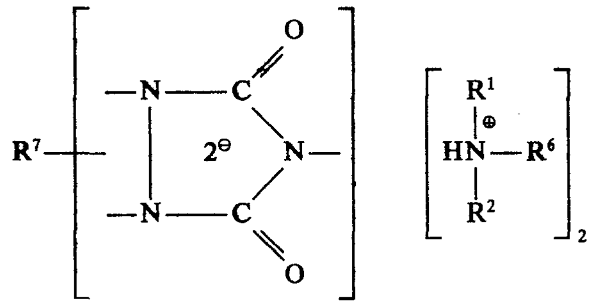

Ia

wherein

$R^1$ and $R^2$ have the meaning described in Claim 1,

$R^6$ represents an aliphatic $C_1-C_{20}$, cycloaliphatic $C_4-C_{15}$, araliphatic $C_7-C_{17}$, or aromatic $C_6-C_{15}$ radical and an aryl or $C_2-C_{12}$ cycloalkyl radical containing hetero atoms such as N, O or S in the ring, which radicals are optionally substituted with halogen, cyano, nitro, hydroxyl, amino, $C_1-C_{20}$ alkyl- or dialkylamino, $C_1-C_{10}$ alkoxy or $C_2-C_{10}$ alkoxycarbonyl, and

$R^7$ has the meaning of $R^4$ or $R^5$.

4. Triazolidine-3,5-dione salts according to Claim 3, characterised in that $R^7$ denotes hydrogen.

5. Triazolidine-3,5-dione salts according to Claim 1 of the general formula II

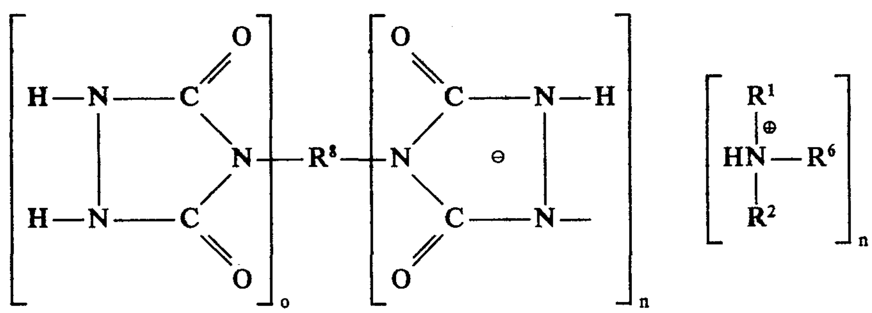

II

wherein

$R^1$, $R^2$ and $R^6$ have the meaning indicated in Claim 4,

$R^8$ represents an (n + o)-valent aliphatic $C_1-C_{20}$ cycloaliphatic $C_4-C_{15}$, araliphatic $C_7-C_{20}$ or aromatic $C_6-C_{15}$ radical optionally substituted with halogen, hydroxyl, $C_1-C_{10}$ alkoxy, $C_1-C_{10}$ alkyl or dialkylamino or $C_2-C_{10}$ alkoxycarbonyl,

n represents an integer from 1 to 5,

o represents 0 or an integer from 1 to 4 and n + o must not be greater than 5.

6. Process for the preparation of triazolidine-3,5-dione salts according to Claims 1 to 5, characterised in that triazolidine-3,5-diones which contain the structure of the general formula III

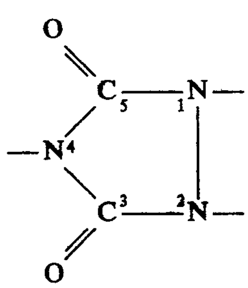

III

at least once and which must contain at least one hydrogen atom bound in the 1-, 2-, or 4-position, are

reacted with amines of the general formula IV

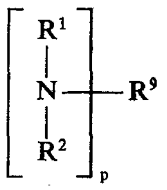

IV

wherein

$R^1$ and $R^2$ have the meaning described in Claim 1,

$R^9$ represents a p-valent aliphatic $C_1-C_{20}$, cycloaliphatic $C_4-C_{15}$, araliphatic $C_7-C_{17}$, or aromatic $C_6-C_{15}$ radical or an aryl or $C_2-C_{12}$ cycloalkyl radical containing hetero atoms such as N, O or S in the ring, which radicals are optionally substituted with halogen, cyano, nitro, hydroxyl, amino, $C_1-C_{10}$ alkylamino, dialkylamino, $C_1-C_{10}$ alkoxy or $C_2-C_{10}$ alkoxycarbonyl, and

p represents an integer from 1 to 5,

the reaction being optionally carried out in water and/or in organic solvents at temperatures of 0 to 150°C.

7. Use of the triazolidine-3,5-dione salts according to Claims 1 to 6 for modifying polymers.

8. Use of the triazolidine-3,5-dione salts according to Claims 1 to 6 for flame-proofing polymers.

**Revendications**

1. Sels de triazolidine-3,5-dione répondant à la formule générale I

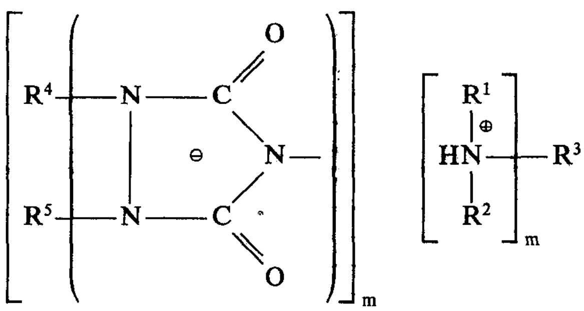

I

dans laquelle:

$R^1$ et $R^2$, identiques ou différents, représentent chacun un atome d'hydrogène, un radical aliphatique en $C_1-C_{10}$, cycloaliphatique en $C_4-C_{10}$, araliphatique en $C_7-C_{17}$ ou aromatique en $C_6-C_{10}$, éventuellement substitués par de l'halogène, un groupe hydroxyle, alcoxy en $C_1-C_{10}$ ou alcoxycarbonyle en $C_2-C_{10}$;

$R^3$ est un radical de valence m, aliphatique en $C_1-C_{20}$, cycloaliphatique en $C_4-C_{15}$, araliphatique en $C_7-C_{17}$, aromatique en $C_6-C_{15}$ ou cycloalkyle en $C_2-C_{12}$ ou aryle, contenant N, O et S, éventuellement substitués par de l'halogène, un groupe cyano, nitro, hydroxyle, amino, alkyl- ou dialkylamino en $C_1-C_{10}$, alcoxy en $C_1-C_{10}$, alcoxycarbonyle en $C_2-C_{10}$, les radicaux $R_1$ et/ou $R_3$ pouvant aussi être reliés ensemble à titre de chaînons d'un radical alkylène cyclique en $C_2-C_{10}$ contenant éventuellement encore N, O ou S dans le cycle,

$R^4$ et $R^5$ identiques ou différents, représentent chacun un atome d'hydrogène, un radical aliphatique en $C_1-C_{20}$, araliphatique en $C_7-C_{17}$, aromatique en $C_6-C_{15}$, éventuellement substitués par de l'halogène, un groupe cyano, hydroxyle, alcoxy en $C_1-C_{10}$ et alcoxycarbonyle en $C_2-C_{10}$, ou un radical alkylcarbonyle en $C_2-C_{20}$ éventuellement substitué par de l'halogène, et

m est un nombre entier valant 1 à 5.

2. Sels de triazolidine-3,5-dione selon la revendication 1, caractérisés en ce que $R_4$ et $R_5$ représentent de l'hydrogène.

3. Sels de triazolidine-3,5-dione selon la revendication 1, de formule générale Ia:

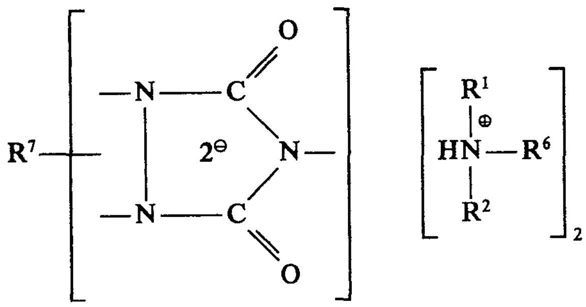

Ia

dans laquelle

$R^1$ et $R^2$ ont le sens décrit dans la revendication 1,

$R^6$ est un radical aliphatique en $C_1-C_{20}$, cycloaliphatique en $C_4-C_{15}$, araliphatique en $C_7-C_{17}$, aromatique en $C_6-C_{15}$ et aryle ou cycloalkyle en $C_2-C_{12}$, contenant dans le cycle des hétéroatomes comme N, O ou S, éventuellement substitués par de l'halogène, un groupe cyano, nitro, hydroxyle, amino, alkyl- ou dialkylamino en $C_1-C_{20}$, alcoxy en $C_1-C_{10}$, alcoxycarbonyle en $C_2-C_{10}$, et

$R_7$ a le sens de $R_4$ ou $R_5$.

4. Sels de triazolidine-3,5-dione selon la revendication 3, caractérisés en ce que $R_7$ représente de l'hydrogène.

5. Sels de triazolidine-3,5-dione selon la revendication 1, de formule générale II:

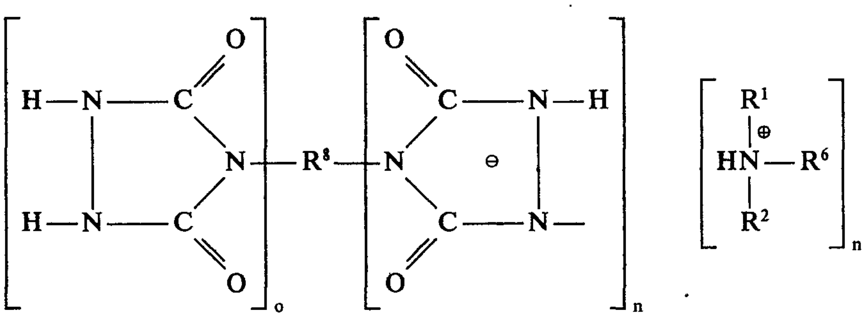

II

dans laquelle

$R^1$, $R^2$ et $R^6$ ont le sens indiqué à la revendication 4,

$R^8$ est un radical de valence (n+o) aliphatique en $C_1-C_{20}$, cycloaliphatique en $C_4-C_{15}$, araliphatique en $C_7-C_{20}$, ou aromatique en $C_6-C_{15}$, éventuellement substitués par de l'halogène, un groupe hydroxyle, alcoxy en $C_1-C_{10}$, alkyl- ou dialkylamino en $C_1-C_{10}$ ou alcoxycarbonyle en $C_2-C_{10}$,

n est un nombre entier valant 1 à 5,

o est nul ou est un nombre entier valant 1 à 4, et n+o ne doit pas être supérieur à 5.

6. Procédé pour préparer des sels de triazolidine-3,5-dione selon les revendications 1 à 5, caractérisé en ce que l'on fait réagir dans de l'eau et/ou dans des solvants organiques, à des températures de 0 à 150° C, des triazolidine-3,5-diones qui contiennent au moins une fois la structure de formule générale III

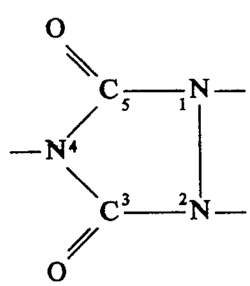

III

et qui doivent contenir au moins un atome d'hydrogène fixé en position 1, 2 ou 4, avec des amines de formule générale IV

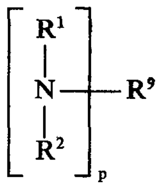

IV

dans laquelle:

$R^1$ et $R^2$ ont le sens indiqué à la revendication 1,

$R^9$ est un radical de valence p, aliphatique en $C_1-C_{20}$, cycloaliphatique en $C_4-C_{15}$, araliphatique en $C_7-C_{17}$, aromatique en $C_6-C_{15}$, éventuellement substitué par de l'halogène, par un groupe cyano, nitro, hydroxyle, amino, alkylamino en $C_1-C_{10}$, dialkylamino, alcoxy en $C_1-C_{10}$, alcoxycarbonyle en $C_2-C_{10}$, ou un radical aryle ou cycloalkyle en $C_2-C_{12}$ contenant dans le cycle des hétéroatomes comme N, O ou S;

p est un nombre entier valant 1 à 5.

7. Utilisation des sels de triazolidine-3,5-dione selon les revendications 1 à 6 pour modifier des polymères.

8. Utilisation des sels de triazolidine-3,5-dione selon les revendications 1 à 6 pour l'ignifugation de polymères.